# EUROPÄISCHE PATENTANMELDUNG

(11) **EP 2 332 587 A1**
(43) Veröffentlichungstag der Anmeldung: **15.06.2011**
(21) Anmeldenummer: 09015399.0
(22) Anmeldetag: 12.12.2009
(51) Int. Cl.: A61L 26/00, A61K 9/00

(54) **Polyurethan-Dispersionen zur Versiegelung von Zitzen der Milchdrüse bei milchliefernden Tieren**

(71) Anmelder: Bayer MaterialScience AG, 51368 Leverkusen (DE)
(72) Erfinder: Die Erfindernennung liegt noch nicht vor

(57) **Zusammenfassung**

Die vorliegende Erfindung betrifft die Verwendung einer wässrigen Polyurethan-Dispersion zur Versiegelung von Zitzen tierischer Milchdrüsen.

## Beschreibung

Die vorliegende Erfindung betrifft die Verwendung einer wässrigen Polyurethan-Dispersion zur Versiegelung von Zitzen tierischer Milchdrüsen.

Um insbesondere während der Trockenstehphase von Kühen das Eindringen von Mikroorganismen zu verhindern, werden die Zitzen von Milchdrüsen temporär versiegelt. Dazu werden heute Lösungen von Polymeren in organischen Lösungsmitteln eingesetzt. Ein solches System ist beispielsweise in der EP 0 973 559 B1 beschrieben.

Die in den bekannten Systemen enthaltenen Lösungsmittel, wie beispielsweise Ethylacetat oder Tetrahydrofuran, gelten jedoch insbesondere bei Kontakt mit Nutztieren, deren Milch für den Verzehr durch Menschen vorgesehen ist als bedenklich, da eine Kontamination der Milch nicht ausgeschlossen werden kann. Weiterhin sind diese Lösungsmittel als "reizend" eingestuft und weisen daher keine gute Hautverträglichkeit auf. Dies führt zumal bei einer Applikationsdauer von mehreren Wochen zu Problemen. Zuletzt gelten die allgemeinen Bedenken gegenüber flüchtigen organischen Lösungsmitteln auch hier, die etwa in der möglichen Umweltgefährdung bestehen.

Aufgabe der Erfindung war daher ein System zur Versiegelung von Zitzen tierischer Milchdrüsen bereit zu stellen, bei dem nicht die Gefahr der Kontamination der Milch besteht, das eine gute Hautverträglichkeit bietet und das unter dem Gesichtspunkt der Umweltgefährdung vorteilhaft ist.

Diese Aufgabe wird erfindungsgemäß durch die Verwendung einer wässrigen Polyurethan-Dispersion zur Versiegelung von Zitzen tierischer Milchdrüsen gelöst.

Wässrige Polyurethan-Dispersionen als Ausgangsbasis zur Herstellung von Versiegelungen weisen verschiedene Vorteile auf. Insbesondere sind sie durch die weitestgehende Abwesenheit von Lösungsmitteln sicher zu verwenden. Eine Absaugung von Lösungsmitteln ist daher nicht erforderlich. Weiterhin ist die Anwendung der wässrigen Polyurethan-Dispersionen im Kontakt mit Tieren und Nahrungsmittel unbedenklich.

Prinzipiell können alle bekannten wässrigen Polyurethan-Dispersionen eingesetzt werden. Bevorzugt sind jedoch anionisch hydrophilierte und anionische/nichtionisch hydrophilierte Polyurethan-Dispersionen.

Besonders bevorzugt einzusetzende Polyurethan-Dispersionen sind erhältlich, indem
A) isocyanatfunktionelle Prepolymere aus
   A1) organischen Polyisocyanaten
   A2) polymeren Polyolen mit zahlenmittleren Molekulargewichten von 400 bis 8000 g/mol, vorzugsweise 400 bis 6000 g/mol und besonders bevorzugt von 600 bis 3000 g/mol, und OH-Funktionalitäten von 1,5 bis 6, bevorzugt 1,8 bis 3, besonders bevorzugt von 1,9 bis 2,1, und
   A3) gegebenenfalls hydroxyfunktionellen Verbindungen mit Molekulargewichten von 62 bis 399 g/mol sowie
   A4) gegebenenfalls isocyanatreaktiven, anionischen oder potentiell anionischen und/oder gegebenenfalls nichtionischen Hydrophilierungsmitteln,
   hergestellt, und
B) dessen freie NCO-Gruppen dann ganz oder teilweise
   B1) gegebenenfalls mit aminofunktionellen Verbindungen mit Molekulargewichten von 32 bis 400 g/mol und
   B2) mit aminofunktionellen, anionischen oder potentiell anionischen Hydrophilierungsmitteln
unter Kettenverlängerung umgesetzt werden und die Prepolymere vor, während oder nach Schritt B) in Wasser dispergiert werden.

Isocyanatreaktive Gruppen sind beispielsweise primäre und sekundäre Aminogruppen, Hydroxygruppen oder Thiolgruppen.

Bevorzugt sind die wässrigen Polyurethan-Dispersionen anionisch mittels Sulfonatgruppen und/oder Carboxylatgruppen hydrophiliert. Besonders bevorzugt sind zur anionischen Hydrophilierung ausschließlich Sulfonatgruppen enthalten.

Um eine gute Sedimentationsstabilität zu erreichen, liegt die zahlenmittlere Teilchengröße der Polyurethan-Dispersionen bevorzugt bei weniger als 750 nm, besonders bevorzugt bei weniger als 500 nm, bestimmt mittels Laserkorrelations-Spektroskopie.

Bevorzugt besitzen die Polyurethan-Dispersionen Feststoffgehalte von 10 bis 70 Gew.-%, besonders bevorzugt von 30 bis 70 Gew.-%, ganz besonders bevorzugt von 30 bis 65 Gew.-% bezogen auf das darin enthaltene Polyurethan.

Weiter bevorzugt weisen diese Polyurethan-Dispersionen weniger als 5 Gew.-%, besonders bevorzugt weniger als 0,2 Gew.-%, bezogen auf die gesamte Dispersionen, an ungebundenen organischen Aminen oder an Ammoniak auf.

Falls gewünscht, kann das Prepolymer A) vor, während oder nach der Dispergierung durch Beimischung einer Base ganz oder teilweise in die anionische Form überführt werden.

Um eine anionische Hydrophilierung zu erreichen müssen in A4) und/oder B2) Hydrophilierungsmittel eingesetzt werden, die wenigstens eine gegenüber NCO-Gruppen reaktive Gruppe wie Amino-, Hydroxy- oder Thiolgruppen aufweisen und darüber hinaus -COO oder -SO₃ oder ― PO₃²⁻ als anionische bzw. deren ganz oder teilweise protonierte Säureformen als potentiell anionische Gruppen aufweisen.

Bevorzugt werden in A4) und/oder B2) solche Verbindungen zur anionischen oder potentiell anionischen Hydrophilierung eingesetzt, die als anionische oder potentiell anionische Funktionalität ausschließlich Sulfonsäure- bzw. Sulfonatgruppen (-SO₃H bzw. -SO₃M, mit M = Alkali- oder Erdalkalimetall) aufweisen.

Geeignete Polyisocyanate der Komponente A1) sind die dem Fachmann an sich bekannten aliphatischen, aromatischen oder cycloaliphatischen Polyisocyanate einer NCO-Funktionalität von größer oder gleich 2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)methane oder deren Mischungen beliebigen Isomerengehalts, 1,4-Cyclohexylendiisocyanat, 4-Isocyanatomethyl-1,8-octandiisocyanat (Nonantriisocyanat), 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3-und/oder 1,4-Bis-(2-isocyanato-prop-2-yl)-benzol (TMXDI), 1,3-Bis(isocyanato-methyl)benzol (XDI) sowie Alkyl-2,6-diisocyanatohexanoate (Lysindiisocyanate) mit C1-C8-Alkylgruppen.

Neben den vorstehend genannten Polyisocyanaten können auch modifizierte Diisocyanate die eine Funktionalität ≥ 2 aufweisen, mit Uretdion-, Isocyanurat-, Urethan-, Allophanat-, Biuret-, Iminooxadiazindion- oder Oxadiazintrionstruktur sowie Mischungen aus diesen auch anteilig eingesetzt werden.

Bevorzugt handelt es sich um Polyisocyanate oder Polyisocyanatgemische der vorstehend genannten Art mit ausschließlich aliphatisch oder cycloaliphatisch gebundenen Isocyanatgruppen oder Mischungen aus diesen und einer mittleren NCO-Funktionalität der Mischung von 2 bis 4, bevorzugt von 2 bis 2,6 und besonders bevorzugt von 2 bis 2,4.

Besonders bevorzugt werden in A1) Hexamethylendiisocyanat, Isophorondiisocyanat oder die isomeren Bis-(4,4'-isocyanatocyclohexyl)methane sowie Mischungen der vorgenannten Diisocyanate eingesetzt.

In A2) werden polymere Polyole mit einem zahlenmittleren Molekulargewicht Mₙ von 400 bis 8000 g/mol, bevorzugt von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Geeignete Polyesterpolyole sind auch die an sich bekannten Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols größer als 2 ist, können zusätzlich auch Monocarbonsäuren wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können in A2) Hydroxylgruppen-aufweisende Polycarbonate, bevorzugt Polycarbonatdiole, mit zahlenmittleren Molekulargewichten Mₙ von 400 bis 8000 g/mol, bevorzugt von 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält die Diolkomponente 40 bis 100 Gew.-% Hexandiol, wobei insbesondere 1,6-Hexandiol und/oder Hexandiolderivate bevorzugt sind. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden.

Hydroxylgruppen aufweisende Polycarbonate sind bevorzugt linear gebaut.

Ebenfalls können in A2) Polyetherpolyole eingesetzt werden.

Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxid und/oder Epichlorhydrin an di- oder polyfunktionelle Startermoleküle. Polyetherpolyole, basierend auf der zumindest anteiligen Addition von Ethylenoxid an di- oder polyfunktionelle Startermoleküle, können auch als Komponente A4) eingesetzt werden (nichtionische Hydrophilierungsmittel).

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

Bevorzugte Komponenten in A2) sind Polytetramethylenglykolpolyether und Polycarbonat-Polyole bzw. deren Mischungen und besonders bevorzugt sind Polytetramethylenglykolpolyether.

In A3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Trimethylolethan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-ε-hydroxy-capronsäureester, ω-Hydroxyhexyl-γ-hydroxybuttersäure-ester, Adipinsäure-(ß-hydroxyethyl)ester oder Terephthalsäurebis(ß-hydroxyethyl)-ester.

Ferner können in A3) auch monofunktionelle isocyanatreaktive Hydroxyl-gruppenhaltige Verbindungen eingesetzt werden. Beispiele solcher monofunktionellen Verbindungen sind Ethanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Geeignete ionisch oder potentiell ionisch hydrophilierende Verbindungen entsprechend der Definition der Komponente A4) sind z.B. Mono- und Dihydroxycarbonsäuren, Mono- und Dihydroxysulfonsäuren, sowie Mono- und Dihydroxyphosphonsäuren und ihre Salze wie Dimethylolpropionsäure, Dimethylolbuttersäure, Hydroxypivalinsäure, Äpfelsäure, Zitronensäure, Glykolsäure, Milchsäure, das propoxylierte Addukt aus 2-Butendiol und NaHSO₃, z.B. beschrieben in der DE-A 2 446 440 (Seite 5-9, Formel I-III).

Geeignete nichtionisch hydrophilierende Verbindungen der Komponente A4) sind z.B. Polyoxyalkylenether, die mindestens eine Hydroxy-, Amino- oder Thiolgruppe enthalten. Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Als Komponente B1) können organische Di- oder Polyamine wie beispielsweise 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 4,4-Diaminodicyclohexylmethan, Hydrazinhydrat, und/oder Dimethylethylendiamin eingesetzt werden.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1- methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin.

Bevorzugt werden 1,2-Ethylendiamin, Bis(4-aminocyclohexyl)methan, 1,4-Diaminobutan, Isophorondiamin, Ethanolamin, Diethanolamin und Diethylentriamin eingesetzt.

Geeignete anionisch hydrophilierende Verbindungen der Komponente B2) sind Alkalimetallsalze der Mono- und Diaminosulfonsäuren. Beispiele solcher anionischen Hydrophilierungsmittel sind Salze der 2-(2-Aminoethylamino)ethansulfonsäure, Ethylendiamin-propyl- oder -butylsulfonsäure, 1,2- oder 1,3-Propylendiamin-ß-ethylsulfonsäure oder Taurin. Weiterhin kann das Salz der Cyclohexylaminopropansulfonsäure (CAPS) aus WO-A 01/88006 als anionisches Hydrophilierungsmittel verwendet werden.

Besonders bevorzugte anionische Hydrophilierungsmittel B2) sind solche, die Sulfonatgruppen als ionische Gruppen und zwei Aminogruppen enthalten, wie die Salze der 2-(2-Aminoethylamino)ethylsulfonsäure und 1,3-Propylendiamin-β-ethylsulfonsäure.

Zur Hydrophilierung können auch Mischungen aus anionischen und nichtionischen Hydrophilierungsmitteln verwendet werden.

In einer bevorzugten Ausführungsform zur Herstellung der Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
55 bis 90 Gew.-% A2),
0,5 bis 20 Gew.-% Summe der Komponenten A3) und B1)
0,1 bis 25 Gew.-% Summe der Komponenten A4) und B2), wobei bezogen auf die Gesamtmengen der Komponenten A1) bis A4) und B1) bis B2) 0,1 bis 5 Gew.-% an anionischen bzw. potentiell anionischen Hydrophilierungsmitteln aus A4) und/oder B2) verwendet werden.

In einer besonders bevorzugten Ausführungsform zur Herstellung der Polyurethan-Dispersionen werden die Komponenten A1) bis A4) und B1) bis B2) in den folgenden Mengen eingesetzt, wobei sich die Einzelmengen stets zu 100 Gew.-% aufaddieren:
5 bis 35 Gew.% Komponente A1),
60 bis 90 Gew.-% A2),
0,5 bis 15 Gew.-% Summe der Komponenten A3) und B1)
0,1 bis 15 Gew.% Summe der Komponenten Komponente A4) und B2), wobei bezogen auf die Gesamtmengen der Komponenten A1) bis A4) und B1) bis B2) 0,2 bis 4 Gew.-% an anionischen bzw. potentiell anionischen Hydrophilierungsmitteln aus A4) und/oder B2) verwendet werden.

In einer ganz besonders bevorzugten Ausführungsform zur Herstellung der Polyurethan-Dispersionen werden die Komponenten A1) bis A4) und B1) bis B2) in den folgenden Mengen eingesetzt, wobei sich die Einzelmengen stets zu 100 Gew.-% aufaddieren:
10 bis 30 Gew.-% Komponente A1),
65 bis 85 Gew.-% A2),
0,5 bis 14 Gew.-% Summe der Komponenten A3) und B1)
0,1 bis 13,5 Gew.-% Summe der Komponenten A4) und B2), wobei bezogen auf die Gesamtmengen der Komponenten A1) bis A4) und B1) bis B2) 0,5 bis 3,0 Gew.-% an anionischen bzw. potentiell anionischen Hydrophilierungsmitteln aus A4) und/oder B2) verwendet werden.

Die Herstellung der Polyurethan-Dispersionen kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z. B. Prepolymer-Mischverfahren, Acetonverfahren oder Schmelzdispergierverfahren verwendet werden. Bevorzugt wird nach dem Aceton-Verfahren verfahren.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösungsmittel wie Aceton, 2-Butanon, die nicht nur zu Beginn der Herstellung, sondern gegebenenfalls in Teilen auch später zugegeben werden können. Bevorzugt sind Aceton und 2-Butanon, besonders bevorzugt ist Aceton. Auch die Zugabe anderer Lösungsmittel ohne isocyanatreaktive Gruppen ist möglich, jedoch nicht bevorzugt.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanat-Gruppen zu isocyanatreaktiven Gruppen im allgemeinen 1,05 bis 3,5, bevorzugt 1,1 bis 3,0, besonders bevorzugt 1,1 bis 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Anschluss wird in einem weiteren Verfahrensschritt, falls noch nicht oder nur teilweise geschehen, das erhaltene Prepolymer mit Hilfe von aliphatischen Ketonen wie Aceton oder 2-Butanon gelöst.

Die Umsetzung der Komponenten a1) bis a4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-methylmorpholin, Methyldiisopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind auch anorganische Basen, wie wässrige Natrium-, Lithium bzw. Kaliumhydroxid einsetzbar.

Bevorzugt sind Natriumhydroxyd, Lithiumhydroxyd oder Kaliumhydroxid, besonders bevorzugt sind Natriumhydroxyd, Lithiumhydroxyd oder Kaliumhydroxid. Ganz besonders bevorzugt sind die Natrium-, Lithium- bzw. Kaliumionen bereits als Kation an anionisch funktionalisierte Bausteine gebunden.

Die Stoffmenge der Basen beträgt im allgemeinen 50 und 125 mol-%, bevorzugt zwischen 70 und 100 mol-% der Stoffmenge der zu neutralisierenden Säuregruppen. Die Neutralisation kann auch gleichzeitig mit der Dispergierung erfolgen, in dem das Dispergierwasser bereits das Neutralisationsmittel enthält.

Bei der Kettenverlängerung in Stufe B) werden NH₂- und/oder NH-funktionelle Komponenten mit den noch verbliebenen Isocyanatgruppen des Prepolymers umgesetzt. Bevorzugt wird die Kettenverlängerung/-terminierung vor der Dispergierung in Wasser durchgeführt.

Geeignete Komponenten zur Kettenverlängerung sind organische Di- oder Polyamine B1) wie beispielsweise Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Diaminodicyclohexylmethan und/oder Dimethylethylendiamin.

Darüber hinaus können auch Verbindungen B1), die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-l-methylaminopropan, 3-Amino-1-ethylaminopropan, 3-Amino-l-cyclohexylaminopropan, 3-Amino-1- methylaminobutan, Alkanolamine wie N-Aminoethylethanolamin, Ethanolamin, 3-Aminopropanol, Neopentanolamin zur Kettenverlängerung bzw. ―terminierung eingesetzt werden.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin verwendet.

Werden zur Kettenverlängerung anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Der Kettenverlängerungsgrad, also das Äquivalentverhältnis von NCO-reaktiven Gruppen der zur Kettenverlängerung und Kettenterminierung eingesetzten Verbindungen zu freien NCO-Gruppen des Prepolymers, liegt im allgemeinen zwischen 40 und 150 %, bevorzugt zwischen 50 und 120 %, besonders bevorzugt zwischen 60 und 120 %.

Die aminischen Komponenten B1) und B2), können gegebenenfalls in verdünnter Form im erfindungsgemäßen Verfahren einzeln oder in Mischungen eingesetzt werden, wobei grundsätzlich jede Reihenfolge der Zugabe möglich ist.

Wenn Wasser als Verdünnungsmittel mit verwendet werden, so beträgt der Verdünnungsmittelgehalt in der in B) eingesetzten Komponente zur Kettenverlängerung bevorzugt 40 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den Polyurethan-Dispersionen beträgt typischerweise weniger als 2 Gew.-%, bevorzugt weniger als 1 Gew.-%, bezogen auf die gesamte Dispersion.

Der pH-Wert der Polyurethan-Dispersionen beträgt typischerweise weniger als 8,0, bevorzugt weniger als 7,5 und liegt besonders bevorzugt zwischen 5,5 und 7,5.

Typischerweise enthalten die Polyurethan-Dispersionen mindestens 10 Gew.-% Polyurethan, bezogen auf den Feststoffanteil aller in der Dispersion enthaltenen filmbildenden Polymere. Bevorzugt sind jedoch mindestens 50 Gew.-%, weiter bevorzugt mindestens 90 Gew.-%, besonders bevorzugt mindestens 95 Gew.-% und insbesondere bevorzugt 100 Gew.-% Polyurethan als filmbildendes Polymer enthalten. Dabei wurde gefunden, dass mit dem Gehalt an Polyurethan die Wasserfestigkeit der erhaltenen Versiegelungen steigt, so dass eine höhere Haltbarkeit gegeben ist.

Wird nicht ausschließlich Polyurethan als filmbildendes Polymer eingesetzt, so können weiterhin andere Polymer-Dispersionen mit eingesetzt werden, z.B. auf Basis von Polyestern, Poly(meth)acrylaten, Polyepoxiden, Polyvinylacetaten, Polyethylen, Polystyrol, Polybutadienen, Polyvinylchlorid und/oder entsprechenden Copolymerisaten.

Die Polyurethan-Dispersionen können neben den Polymer-Dispersionen zusätzlich auch Hilfs- und Zusatzstoffe enthalten. Beispiele für solche Hilfs- und Zusatzstoffe sind Vernetzer, Verdicker, Thixotropiermittel, Stabilisatoren, Antioxidantien, Lichtschutzmittel, Emulgatoren, Tenside, Weichmacher, Pigmente, Füllstoffe und Verlaufshilfsmittel.

Der Auftrag der erfindungsgemäßen Polyurethan-Dispersionen kann nach allen an sich bekannten Applikationsformen erfolgen, genannt seien beispielsweise Tauchen, Streichen, Gießen oder Sprühen. Besonders bevorzugt sind Sprühen und Tauchen, ganz besonders bevorzugt ist Tauchen.

Die Polyurethan-Dispersionen werden üblicherweise an der Luft getrocknet.

Ein mehrschichtiger Auftrag mit gegebenenfalls zwischengelagerten Trocknungsschritten ist grundsätzlich auch möglich.

Die nach dem Trocknen erhaltenen Filme haben typischerweise eine Dicke von 0,1 bis 1500 µm, bevorzugt 1 bis 500 µm, besonders bevorzugt 5 bis 200 µm, ganz besonders bevorzugt 50 bis 150 µm.

Weiterhin kann die Polyurethan-Dispersionen mit Wirkstoffen versetzt werden oder in Kombination mit Wirkstoffen aufgebracht werden. Im Folgenden werden Wirkstoffe definiert als Elemente oder chemische Verbindungen, die eine Wirkung auf lebende Systeme, insbesondere Prionen, Viren, Bakterien, Zellen, Pilze und Organismen, haben.

Beispiele sind biozide Wirkstoffe, die z.B. pestizid, fungizid, algizid, insektizid, herbizid, spermizid, parasitizid, antibakteriell (Bakterien vernichtend), bakteriostatisch, antibiotisch, antimykotisch (Pilze vernichtend); antiviral (Viren vernichtend), virostatisch und/oder antimikrobiell (Mikroben vernichtend) wirken. Auch Wirkstoffkombinationen und die Kombination beispielsweise mit Hilfsmitteln, Bindemitteln, Neutralisationsmitteln oder Additiven sind möglich. Auch andere Wirkstoffe und Kombinationen, beispielsweise Wirkstoffe aus dem Bereich der Humanmedizin bzw. Tiermedizin sind einsetzbar. Bevorzugt wird zumindest ein antibakteriell, bakteriostatisch oder antibiotisch wirkendes Additiv zugesetzt.

### Beispiele

Sofern nicht abweichend gekennzeichnet, beziehen sich alle Prozentangaben auf das Gewicht.

Sofern nicht abweichend vermerkt, beziehen alle analytischen Messungen auf Temperaturen von 23°C.

Die Bestimmung der Festkörpergehalte erfolgte nach DIN-EN ISO 3251.

NCO-Gehalte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23°C mit einem Rotationsviskosimeter der Firma Anton Paar Germany GmbH, Ostfidern, DE bestimmt.

Die Bestimmung der mittleren Teilchengrößen (angegeben ist das Zahlenmittel) der Polyurethan-Dispersionen erfolgte mittels Laserkorrelations-Spektroskopie (Gerät: Malvern Zetasizer 1000, Malver Inst. Limited).

### Verwendete Substanzen und Abkürzungen:

| | |
|---|---|
| Diaminosulfonat: | NH₂-CH₂CH₂-NH-CH₂CH₂-SO₃Na (45 %ig in Wasser) |
| Desmophen^{®} 2020/C2200: | Polycarbonatpolyol, OH-Zahl 56 mg KOH/g, zahlenmittleres Mo-lekulargewicht 2000 g/mol (Bayer MaterialScience AG, Leverku-sen, DE) |
| PolyTHF^{®} 2000: | Polytetramethylenglykolpolyol, OH-Zahl 56 mg KOH/g, zahlen-mittleres Molekulargewicht 2000 g/mol (BASF AG, Ludwigsha-fen, DE) |
| PolyTHF^{®} 1000: | Polytetramethylenglykolpolyol, OH-Zahl 112 mg KOH/g, zahlen-mittleres zahlenmittleres Molekulargewicht 1000 g/mol (BASF AG, Ludwigshafen, DE) |
| Polyether LB 25: | monofunktioneller Polyether auf Ethylenoxid-/Propylenoxidbasis zahlenmittleres Molekulargewicht 2250 g/mol, OH-Zahl 25 mg KOH/g (Bayer MaterialScience AG, Leverkusen, DE) |

### Beispiel 1: Polyurethan-Dispersion 1

987,0 g PolyTHF^{®} 2000, 375,4 g PolyTHF^{®} 1000, 761,3 g Desmophen® C2200 und 44,3 g Polyether LB 25 wurden in einer Standard-Rührapparatur auf 70°C aufgeheizt. Anschließend wurde bei 70°C innerhalb von 5 min ein Gemisch aus 237,0 g Hexamethylendiisocyanat und 313,2 g Isophorondiisocyanat zugegeben und bei 120°C gerührt, bis der theoretische NCO-Wert erreicht war. Das fertige Prepolymer wurde mit 4830 g Aceton gelöst und dabei auf 50°C abgekühlt und anschließend wurde eine Lösung aus 25,1 g Ethylendiamin, 116,5 g Isophorondiamin, 61,7 g Diaminosulfonat und 1030 g Wasser innerhalb von 10 min zudosiert. Die Nachrührzeit betrug 10 min. Dann wurde durch Zugabe von 1250 g Wasser dispergiert. Es folgte die Entfernung des Lösemittels durch Destillation im Vakuum. Der Restgehalt an Aceton lag bei unter 1 Gew.-% bezogen auf. die fertige Dispersion.

Die erhaltene weiße Dispersion hatte nachfolgenden Eigenschaften:

| | |
|---|---|
| Feststoffgehalt: | 61 % |
| Partikelgröße (LKS): | 312 nm |
| Viskosität (Viskosimeter, 23°C): | 241 mPas |
| pH (23°C): | 6,02 |

### Beispiel 2: Polyurethan-Dispersion 2

450 g PolyTHF^{®} 1000 und 2100 g PolyTHF^{®} 2000 wurden auf 70°C aufgeheizt. Anschließend wurde bei 70°C innerhalb von 5 min ein Gemisch aus 225,8 g Hexamethylendiisocyanat und 298,4 g Isophorondiisocyanat zugegeben und solange bei 100-115°C gerührt bis der theoretische NCO-Wert unterschritten war. Das fertige Prepolymer wurde mit 5460 g Aceton bei 50°C gelöst und anschließend eine Lösung aus 29,5 g Ethylendiamin, 143,2 g Diaminosulfonat und 610 g Wasser innerhalb von 10 min zudosiert. Die Nachrührzeit betrug 15 min. Danach wurde innerhalb von 10 min durch Zugabe von 1880 g Wasser dispergiert. Es folgte die Entfernung des Lösemittels durch Destillation im Vakuum und es wurde eine lagerstabile Dispersion erhalten. Der Restgehalt an Aceton lag bei unter 1 Gew.-% bezogen auf. die fertige Dispersion.

| | |
|---|---|
| Feststoffgehalt: | 56 % |
| Partikelgröße (LKS): | 276 nm |
| Viskosität: | 1000 mPas |
| pH (23°C): | 7,15 |

### Beispiel 3: Polyurethan-Dispersion 3

1649,0 g eines Polyesters aus Adipinsäure, Hexandiol und Neopentylglykol mit einem mittleren Molekulargewicht von 1700 g/mol wurden auf 65°C aufgeheizt. Anschließend wurden bei 70°C innerhalb von 5 min 291,7 g Hexamethylendiisocyanat zugegeben und solange bei 100-115°C gerührt bis der theoretische NCO-Wert unterschritten war. Das fertige Prepolymer wurde mit 3450 g Aceton bei 50°C gelöst und anschließend eine Lösung aus 16,8 g Ethylendiamin, 109,7 g Diaminosulfonat und 425 g Wasser innerhalb von 3 min zudosiert. Die Nachrührzeit betrug 15 min. Danach wurde innerhalb von 10 min durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 42 % |
| Partikelgröße (LKS): | 168 nm |
| Viskosität: | 425 mPas |
| pH-Wert: | 7,07 |

### Beispiel 4: PUR-Dispersion 4

82,5 g PolyTHF^{®} 1000, 308 g PolyTHF^{®} 2000 und 10,0 g 2-Ethylhexanol wurden auf 70°C aufgeheizt. Anschließend wurde bei 70°C innerhalb von 5 min ein Gemisch aus 41,4 g Hexamethylendiisocyanat und 54,7g Isophorondiisocyanat zugegeben und solange bei 110-125°C gerührt bis der theoretische NCO-Wert unterschritten war. Das fertige Prepolymer wurde mit 880 g Aceton bei 50°C gelöst und anschließend eine Lösung aus 3,8 g Ethylendiamin, 4,6 g Isophorondiamin, 26,3 g Diaminosulfonat und 138 g Wasser innerhalb von 10 min zudosiert. Die Nachrührzeit betrug 15 min. Danach wurde innerhalb von 10 min durch Zugabe von 364 g Wasser dispergiert. Es folgte die Entfernung des Lösemittels durch Destillation im Vakuum und es wurde eine lagerstabile Dispersion erhalten.

| | |
|---|---|
| Feststoffgehalt: | 49 % |
| Partikelgröße (LKS): | 181 nm |
| Viskosität: | 1300 mPas |
| pH-Wert: | 7,22 |

### Beispiel 5: Herstellung der Beschichtungslösungen

Die in den Beispielen 1 bis 4 hergestellten PUR-Dispersionen wurden jeweils mit Wasser und/oder Verdicker (Borchigel ALA) auf eine Viskosität von 300 bis 500 mPas (23 °C) eingestellt und mit blauer Lebensmittelfarbe (Dualcert Blau Nr. 1, SENSIENT COLORS, UK) eingefärbt.

### Beispiel 6: Versiegelung von Zitzen

Die Zitzenversiegelungsexperimente wurden an Milchkühen während der hormonell induzierten Phase der Trockenstellung durchgeführt. Dazu wurde jeweils eine der im Beispiel 5 hergestellten Beschichtungslösungen flächig durch Dippen auf die Zitzen einer Milchdrüse einer Kuh aufgebracht. Die Trocknung erfolgte an der Luft. Die erhaltenen Beschichtungen versiegelten die Zitzen. Sie wiesen darüber hinaus überraschenderweise auch unter den feuchten Bedingungen in einem Kuhstall eine Haltbarkeit von bis zu 3 Tagen auf, d.h. innerhalb dieses Zeitraums blieb die Versiegelung erhalten. Damit konnten die erfindungsgemäßen Systeme mit marktüblichen lösemittelhaltigen Systemen wie z.B. DryFlex von DeLaval oder calgodip T-Hexx Dry von Kleancare Hygiene GmbH mithalten, wobei sie im Gegensatz zu den marktüblichen Systemen ohne (flüchtige) organische Lösungsmittel auskommen.

## Patentansprüche

1. Verwendung einer wässrigen Polyurethan-Dispersion zur Versiegelung von Zitzen tierischer Milchdrüsen.

2. Verwendung nach Anspruch 1, **dadurch gekennzeichnet, dass** die Polyurethan-Dispersion anionisch und/oder nichtionisch hyrophiliert ist.

3. Verwendung nach einem der Ansprüche 1 oder 2, **dadurch gekennzeichnet, dass** die Polyurethan-Dispersion erhältlich ist, indem
A) isocyanatfunktionelle Prepolymere aus
A1) organischen Polyisocyanaten
A2) polymeren Polyolen mit zahlenmittleren Molekulargewichten von 400 bis 8000 g/mol, vorzugsweise von 400 bis 6000 g/mol und besonders bevorzugt von 600 bis 3000 g/mol, und OH-Funktionalitäten von 1,5 bis 6, bevorzugt von 1,8 bis 3, besonders bevorzugt von 1,9 bis 2,1, und
A3) gegebenenfalls hydroxyfunktionellen Verbindungen mit Molekulargewichten von 62 bis 399 g/mol sowie
A4) gegebenenfalls isocyanatreaktiven, anionischen oder potentiell anionischen und/oder gegebenenfalls nichtionischen Hydrophilierungsmitteln,
hergestellt werden, und
B) deren freie NCO-Gruppen dann ganz oder teilweise
B1) gegebenenfalls mit aminofunktionellen Verbindungen mit Molekulargewichten von 32 bis 400 g/mol und
B2) mit aminofunktionellen, anionischen oder potentiell anionischen Hydrophilierungsmitteln
unter Kettenverlängerung umgesetzt werden und die Prepolymere vor, während oder nach Schritt B) in Wasser dispergiert werden.

4. Verwendung nach Anspruch 3, **dadurch gekennzeichnet, dass** die Komponenten A1) bis A4) und B1) bis B2) in den folgenden Mengen eingesetzt werden, wobei sich die Einzelmengen stets zu 100 Gew.-% addieren:
5 bis 40 Gew.-% Komponente A1),
55 bis 90 Gew.-% A2),
0,5 bis 20 Gew.-% Summe der Komponenten A3) und B1)
0,1 bis 25 Gew.-% Summe der Komponenten A4) und B2),
wobei bezogen auf die Gesamtmengen der Komponenten A1) bis A4) und B1) bis B2) 0,1 bis 5 Gew.-% an anionischen bzw. potentiell anionischen Hydrophilierungsmitteln aus A4) und/oder B2) verwendet werden.

5. Verwendung nach einem der Ansprüche 1 bis 4, **dadurch gekennzeichnet, dass** die mittels Laserkorrelations-Spektroskopie bestimmte zahlenmittlere Teilchengröße der Teilchen in der Polyurethan-Dispersionen weniger als 750 nm, bevorzugt weniger als 500 nm beträgt.

6. Verwendung nach einem der Ansprüche 1 bis 5, **dadurch gekennzeichnet, dass** die Polyurethan-Dispersion Feststoffgehalte von 10 bis 70 Gew.-%, besonders bevorzugt von 30 bis 70 Gew.-%, ganz besonders bevorzugt von 30 bis 65 Gew.-% bezogen auf das darin enthaltene Polyurethan aufweist.

7. Verwendung nach einem der Ansprüche 1 bis 6, **dadurch gekennzeichnet, dass** die Polyurethan-Dispersionen weniger als 5 Gew.-%, besonders bevorzugt weniger als 0,2 Gew.-%, bezogen auf die gesamte Dispersionen, an ungebundenen organischen Aminen enthält.

8. Verwendung nach einem der Ansprüche 1 bis 7, **dadurch gekennzeichnet, dass** in der Polyurethan-Dispersion keine flüchtigen Amine und kein Ammoniak enthalten sind.

9. Verwendung nach einem der Ansprüche 1 bis 8, **dadurch gekennzeichnet, dass** in der Polyurethan-Dispersion weniger als 2 Gew.-%, bevorzugt weniger als 1,5 Gew-%, weiter bevorzugt weniger als 1 Gew-% und insbesondere weniger als 0,5 Gew.-% organische Lösungsmittel enthalten sind.

10. Verwendung nach einem der Ansprüche 1 bis 9, **dadurch gekennzeichnet, dass** in der Polyurethan-Dispersion zumindest ein biozider Wirkstoff enthalten ist.

11. Verwendung nach Anspruch 10, **dadurch gekennzeichnet, dass** der biozide Wirkstoff. pestizid, fungizid, algizid, insektizid, herbizid, spermizid, parasitizid, antibakteriell, bakteriostatisch, antibiotisch, antimykotisch; antiviral, virostatisch und/oder antimikrobiell ist.

12. Verwendung nach einem der Ansprüche 1 bis 11, **dadurch gekennzeichnet, dass** die Beschichtung auf Zitzen der Milchdrüse von Milchkühen aufgebracht wird.

13. Verwendung nach Anspruch 12 **dadurch gekennzeichnet, dass** die Polyurethan-Dispersion während der Trockenstehphase der Milchkühe auf die Zitzen aufgebracht wird.
